Europäisches Patentamt

European Patent Office

Office européen des brevets

(11) Publication number: **0 353 045 B1**

# EUROPEAN PATENT SPECIFICATION

(45) Date of publication of patent specification: **15.11.95**

(51) Int. Cl.⁶: **A61K 38/27**, A61K 9/16, A61K 9/22, A61K 9/52

(21) Application number: **89307593.7**

(22) Date of filing: **26.07.89**

(54) **Devices and methods for growth promotion in poultry roasters.**

(30) Priority: **26.07.88 US 224521**
**25.07.89 US 382834**

(43) Date of publication of application:
**31.01.90 Bulletin 90/05**

(45) Publication of the grant of the patent:
**15.11.95 Bulletin 95/46**

(84) Designated Contracting States:
**AT BE CH DE ES FR GB GR IT LI LU NL SE**

(56) References cited:
**EP-A- 0 192 360      EP-A- 0 210 039**
**EP-A- 0 246 540      EP-A- 0 257 368**
**EP-A- 0 283 458      WO-A-87/06828**
**US-A- 4 452 775**

**ENDOCRINOLOGY, vol 118, No. 5, 1986, pp. 1961-1965, THE ENDOCRINE SOCIETY; F.C. LEUNG et al.: "Purified chicken growth hormone (GH) and a human pancreatic GH-Releasing hormone increase body weight gain in chickens"**

(73) Proprietor: **Rutgers, The State University of New Jersey**

**New Brunswick**
**New Jersey 08900 (US)**

Proprietor: **AMERICAN CYANAMID COMPANY**
**One Cyanamid Plaza**
**Wayne, NJ 07470-8426 (US)**

(72) Inventor: **Scanes, Colin G.**
**1601 Van Buren Drive**
**North Brunswick, New Jersey 08902 (US)**
Inventor: **Ricks, Catherine A.**
**8 Upton Lane**
**Yardley, Pennsylvania 19067 (US)**

(74) Representative: **Watkins, Arnold Jack et al**
**European Patent Attorney**
**Frank B. Dehn & Co.**
**Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

ENDOCRINOLOGY, vol.120, No. 2, 1987, pp. 651-658, THE ENDOCRINE SOCIETY; W.H. BURKE et al.: "The properties of recombinant chicken growth hormone and its effects on growth, body composition, feed efficiency, and other factors in broiler chickens"

**Description**

This invention relates to a method for increasing the growth rate or feed efficiency in poultry roasters (hereinafter referred to at times as "roasters"). Also provided are devices which are adapted to provide continuous infusion of recombinant chicken somatotropin or other effective somatotropins.

It is desired in the roaster producing art to provide increased rate of growth and efficiency of feed utilization. Such a process would permit increase in the productivity by the grower of roasters such as chicken roasters, by decreasing the time which is required to produce roasters of a particularly desired weight. The grower on an annual basis would be able to provide more roasters of such weight without increasing facilities and the labor required in caring for the roasters. Such a process would increase the amount of roaster meat available for human consumption.

Considerable attention has been given to the biological activity of polypeptide compounds referred to as somatotropins or growth hormones.

On the basis of previously reported experimentation, it was concluded that recombinant chicken somatotropin when administered to broiler chickens from age 2-24 days by three times per day injection had little or no effect on growth or feed consumption in broiler chicks. (W. H. Burke et al., Endocrinology 120: 651-658, 1987. Reference is also made to the article Effect of Pattern of Administration on the Response to Exogenous, Pituitary-Delivered Chicken Growth Hormone by Broiler-Strain Pullets, R. Vasilatos-Youken et al., General And Comparative Endocrinology 71, 268-283 (1988)).

It has surprisingly been found in contrast to the findings above referred to in the Burke et al. reference that administration of an effective avian somatotropin to roasters provides a substantial increase in their growth rate or feed efficiency. It has been further found that administration of an effective amount of a suitable somatotropin by continuous parenteral infusion is a desirable manner to carry out the invention. An effective amount of a somatotropin is suitably at least about 1 microgram/kg/day administered by continuous infusion to roasters such as chicken, turkey, duck and goose roasters. A suitable range of somatotropin administration is within the range of about 1 to about 250 micrograms/kg/day. A presently preferred amount of somatotropin administration is at least about 5 micrograms/kg/day 5 and 50 micrograms/kg/day has been found to be a suitable amount when administered continuously by infusion for an effective period to attain a desired promotion of growth, such as for at least about 2 weeks or for the last 3 or 4 weeks of the growth period being suitable.

A somatotropin which has been found to be highly suitable for use in carrying out the process in chicken roasters is recombinant chicken somatotropin.

It has been found highly functional to use a device for continuously administering an effective somatotropin which comprises an osmotic pump implant containing in solution an amount of somatotropin using a sterile and biological acceptable solution adequate for effective continuous infusion for several days during the growing period as desired, for example, for at least about 21 days. Other suitable implants and administrations can be used which contain an effective somatotropin and which preferably continuously release for systemic absorption somatotropin for a desired growth period.

The implants are suitably inserted subcutaneously using good practices known to those skilled in the roaster producing art.

In the accompanying drawings,

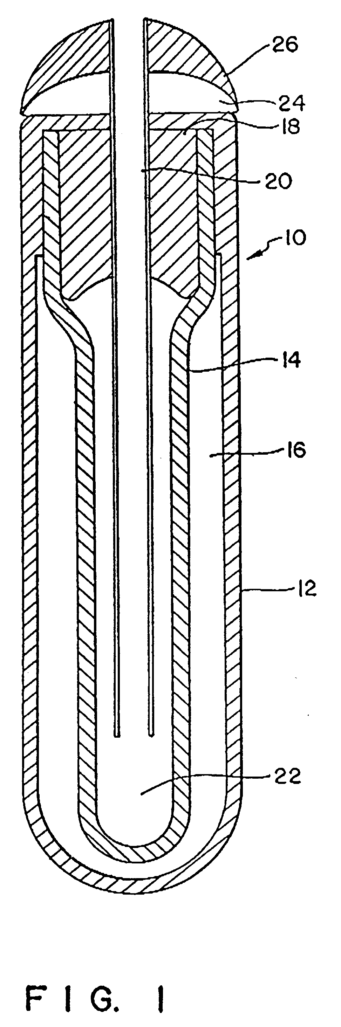

FIG. 1 shows a cross section of an osmotic pump implant adapted for insertion into a suitable subcutaneous region of a roaster and adapted for continuous release of a contained solution of somatotropin at a preselected rate.

The somatotropins which can be used for carrying out this invention include known polypeptide compounds. They can be compounds which occur naturally and which are secreted by the pituitary gland of animals, including chickens, turkeys, ducks and other poultry, used as a protein source for man.

Somatotropin found suitable in carrying out this invention is made using recombinant DNA technology. The cGH gene is cloned using known techniques such as described and referred to by W. H. Burke et al., cited above. Effective natural somatotropin of poultry or other animals can be used but is difficult to obtain and expensive in view of the small amount present in the respective pituitary glands.

Other somatotropins can be used which are obtained by known cloning DNA techniques and using GH genes of other species. The amino acid sequences of somatotropins derived from the different species vary to some degree. Notwithstanding the variance of the chemical structures, some of these somatotropins will show the bioactivity required in carrying out the invention.

Additionally, there will be a number of effective variants, constructs, segments, derivatives and the like of recombinant chicken somatotropin or other somatotropins such as other recombinant DNA derived somatotropins. Certain segments of the somatotropin molecule contribute the biological activity required in carrying out this invention. Insofar as such variants, constructs, segments and derivatives and the like have the essential biological activity for carrying out this invention, they are meant to be included within the term "effective somatotropin".

Derivatives of somatotropins which can be used in carrying out this invention can be selected from effective and biocompatible derivatives, such as carboxymethyl, methyl sulfide, hydroxysuccinic, carbamidomethylated and the like derivatives.

The effective somatotropin such as recombinant somatotropin is desirably dissolved in a sterile and isotonic aqueous solution. The solution can be suitably buffered with a biocompatible buffer. The solution will be made at a somatotropin concentration appropriate for the particular form of the administration used. Ordinarily, using the skill within the art, the concentration will be within the range of about 1 to 10 percent, depending upon the somatotropin used, the particular dosage form selected, the daily dose selected and other factors pertaining in formulating the dosage forms.

The dosage forms suitable for the administration to roasters used in carrying out the invention are bioacceptable subcutaneous forms. The dosage forms can be implants inserted subcutaneously at the beginning of the growth promoting process and desirably provide a continuous daily dosage amount of an effective somatotropin to the roasters in such manner as to be absorbed systemically.

Effective somatotropins can be suitably administered by injection in the form of biologically active parenteral compositions. Among the parenteral compositions useful for administration of somatotropins are gels, pastes, microspheres, microcapsules, implants and the like. As such, there is considerable interest in providing dosage forms of biologically active substances which release the substance in a controlled manner and thus, reduce the frequency of administration.

The preparation of sustained release compositions of somatotropins require attention to special problems due to their complex modes of action and intricate structures of the macromolecules.

The compositions useful for this type of administration can be prepared by dissolving a modified or derivatized somatotropin in dilute ammonium hydroxide and then adding a solution of an alkali metal benzoate, laurate, carbonate or the like thereto. A nonionic surfactant is thereafter admixed with the solution and the resulting mixture spray dried. The thus formed solids are then admixed with molten fat or wax or a mixture thereof and the resulting molten mixture sprayed through an air/liquid spray nozzle equipped with a heated jacket to maintain the incoming air and the molten phase at a temperature above the melting point. The microspheres are formed as the molten droplets cool. These are collected on a series of sieves in the desired size range of about 45 to 180 microns and retained for use. Microspheres which are not of the desired size range are recycled. Alternatively, the homogeneous mixture is fed onto a centrifugal disc and the microspheres thus formed collected as above, or the molten mixture are cooled and milled to the desired average particle size range.

The biologically active microspheres are then dispersed in a pharmaceutically and pharmacologically acceptable liquid vehicle for parenteral injection. The microsphere-liquid composition is generally administered by subcutaneous injection under the skin of the animal usually in the vicinity of the head and neck.

Modified or derivatized somatotropins also are prepared as biocompatible implants which are injected under the skin of the animal using a conventional pellet implant gun. These compositions are prepared by admixing a powdered modified or derivatized somatotropin with a wax such as castor wax or with a mixture of a copoly (glycolide/lactide), magnesium hydroxide, and a condensate of ethylene oxide prepared with a hydrophobic base formed by condensation of propylene oxide with propylene glycol. The thus formed compositions are then introduced into a pelleting press and formed into cylindrical pellets about 25.4/8 mm (1/8 inch) in diameter. The thus formed pellets are administered with a conventional pellet implant gun.

One dosage form found to function in a highly effective manner is a subcutaneous device in the form of an osmotic pump implant. FIG. 1 shows a cross section of such an osmotic pump implant 10. The osmotic pump implant has an exterior wall 12, which is made using a suitable material and acts as a semipermeable membrane. Positioned within the vessel formed by exterior wall 12 is an interior vessel 14 which is positioned in fixed and spaced relationship with the vessel formed by exterior wall 12. The upper wall of inner vessel 14 extends outwardly to make intimate contact with the exterior wall 12. In the enclosed space 16 formed by the contact between the exterior wall 12 and the interior vessel 14 is an osmotic agent region 16, which is filled with a suitable osmotic agent. The tope of the interior vessel 14 is filled with a plug 18 which has a hole therethrough through which is placed a suitable conduit tube 20, which communicates between the reservoir 22 holding the somatotropin solution and the subcutaneous region of the roasters into which the osmotic pump implant is placed. The osmotic pump implant has a top flange 24 fitted to the conduit tube 20, which moderates the flow of the somatotropin solution continuously at a predetermined rate into the subcutaneous region of the treated roaster. The osmotic pump plant has a removable cap 26 to permit the continuous dosage flow of somatotropin when the osmotic pump implant is inserted into the subcutaneous region of the roasters.

The osmotic pumps are desirably treated prior to use to reduce adsorption or binding of proteins or peptides to the inner walls or surfaces of the pump. This pretreatment can be done in accordance with the recommendations of the pump manufacturer: the pump is filled with 1 percent organophosphosilane for 72 hours, the organophosphosilane is removed and the pump is dried. The vehicle for somatotropin (chicken somatotropin) used was saline, pH 8.4, containing 1 percent bovine serum albumin (Sigma, St. Louis, MO). The bovine serum albumin was added to reduce peptide or protein binding to the internal walls and surfaces of the pump.

The osmotic pump operates by the slow infusion of biological fluid of the subcutaneous region of the implanted roaster through the exterior wall 12. The incoming fluid causes gradual expansion of the osmotic agent filling the enclosure region 16. As the osmotic agent expands, a slow, continuously increasing pressure is exerted upon the exterior side walls of the interior vessel 14, thereby forcing at a predetermined rate the somatotropin into the subcutaneous region of the roaster being treated. More than one of the osmotic pump implants can be inserted to obtain an increase in daily dosage of the contained somatotropin or the implant can be modified to deliver a higher daily dose. The osmotic agent is selected to obtain the desired flow rate to provide the daily continuous dosage desired. The concentration of the somatotropin, the osmotic agent, the respective volumes of the osmotic agent region, the internal vessel, the exterior wall and the configuration of the communication tube will be varied to provide the desired flow and length of treatment.

The somatotropin solution is placed into the internal vessel employing sterile conditions and appropriate procedures followed by those skilled in the art of forming such sterile, implantable dosage forms. The osmotic pump implants after filling are sealed and appropriately packaged and stored until they are to be used for implantation to carry out this growth promotion invention.

It has been found that suitable osmotic pump implants are sold under the designation Alzet by Alza Corporation.

Other suitable dosage implant forms can be used for insertion subcutaneously. The sterile solution of somatotropin can be compounded with a suitable biocompatible material to form an encapsulated dosage form. Alternatively, the somatotropin can be suitably intermixed with a bioacceptable material to form an implant as by tableting or the like.

The somatotropin is administered to the roasters so as to be absorbed systemically. A suitable manner of administration is to insert subcutaneously a solid dosage form. The solid dosage form is referred to as an implant. The implant can be inserted in any suitable subcutaneous portion of the roaster. A suitable place has been found to be the convention location of the nape of the neck or above the scapula.

The implant can be in any biocompatible geometric pellet form, such as a cylinder, tablet, sphere or the like. It is desirable that the implant be free of sharp edges or points. Implants with such sharp edges or points can fail to be bioacceptable to the roaster treated.

It has been found suitable to disperse the somatotropin in a suitable polymer and form a pellet thereof, following technique known in the art.

An implant can be made using an insert core as a base for coating thereon a mixture of somatotropin and an extending agent such as a suitable polymer, wax, fatty substance, or the like, for example, a low melting beeswax, peanut oil-aluminum monostearate, slightly soluble or partially soluble polymers, or other suitable materials. The amount or concentration of the somatotropin will be adjusted to provide the desired daily dosages diffusing from the subcutaneous implants. The coating can alternatively be made by using a suitable polymer with a slow dissolution rate permitting a regulated release to daily dosages. In such an instance, a core can be employed which is biosoluble. In such implants, the exposed biosoluble and biocompatible core will dissolve and disappear.

Reference is made for assistance in making suitable implants to U.S. Patents Nos. 2,413,419; 2,895,875; 3,428,729; 3,830,907; 3,857,932; 4,096,239; and 4,191,741.

Administration of somatotropin can be made in other suitable ways known or developed by the art in order to provide effective systemic administration of somatotropin in accordance with this invention.

In regard to chickens, they are selected for carrying out the methods of this invention which are of suitable age, weight and breeding. It has been found that chickens of a heavy breed such as Hubbard x Hubbard are suitable. Other chicken breeds and varieties can also be used. Chickens used will be older chickens, 4-5 weeks old or more. It has been found desirable to use chickens which are about 6 weeks of age or older to be suitable to provide chickens roasters by this invention. Chickens used in the invention will ordinarily weigh at least 1.3 to 1.76 pounds (about 0.6 to 0.8 kg). It has been found that chickens weighing at least about 3 or 4 pounds (about 1.4 to 1.8 kg) to be suitable. Chickens of about 7 to about 12 weeks of age can be selected to carry out the invention.

Instead of using chickens in carrying out the roaster growth promoting method of this invention, other poultry used to provide meat for human consumption can be used, such as for example turkeys, ducks and geese. Corresponding roasters are selected for carrying out the methods of this invention. The time of administration of effective somatotropin can vary somewhat depending on the species of poultry used. For example, the time of administration in the case of turkeys can be somewhat later than in chickens. Any adjustment in the time of administration of the effective somatotropin will be made using the skill of the art, the description herein of the invention and the knowledge of the respective life cycles of the pertinent poultry species.

Suitable chickens selected, for example, in carrying out the invention will be treated individually by administration of an effective somatotropin. Generally speaking, the administration can be by insertion of an implant containing an effective somatotropin in the subcutaneous region of the roaster. The implant will preferably be a type which will provide continuous infusion of the somatotropin as desired to provide the growth promotion in the roasters. Alternatively, the effective somatotropin can be administered parenterally, e.g. subcutaneously, in the form of a suitable sustained release formulation, described herein. The dosage forms will be inserted or administered usually at least 2 or 3 weeks before the chicken roasters are slaughtered and dressed for human consumption. The time of insertion of the subcutaneous implant will vary upon various factors such as the age of the roasters selected, the final weight desired in the roasters, and other factors.

The amount of somatotropin administered per day/kg weight of the roasters can vary depending upon the increase in growth desired and other factors. Ordinarily, an effective amount will be administered of at least 1 microgram/kg wt/day up to about 250 micrograms/kg wt/day, desirably at least about 5 micrograms/kg wt/day, and preferably about 50 micrograms or more/kg wt/day.

The roasters will be grown using good growing practices known and followed by those skilled in the roaster growing art, including good hygiene practice, fresh water supply and a balanced diet, preferably having a high protein content, and water made available on an ad libitum basis.

A grain feed for the roasters will preferably be supplemented by adding thereto a high protein source. Suitable high protein supplement can be selected from soybean meal, fish meal, cotton seed meal, commerical animal source protein products, lysine, methionine, and the like and combinations thereof. Commercial mixed feeds for poultry having suitable high protein content are available commercially, which are designed for high growth rates.

By the following methods of this invention, it has been found that a substantial increase in muscle mass can be obtained, for example, a substantial increase in pectoralis (breast) muscle mass.

The following Examples are meant to be illustrative and not limiting.

Certain modifications of the methods and compositions of this invention as above described herein will be suggested to those in the art of growing roasters and will come within the scope of this invention.

Example 1

ANIMALS

Twelve week old male heavy strain chickens (Hubbard x Hubbard, Avian Services, Frenchtown, New Jersey) were assigned to three experimental groups that were averaged for starting weights. Mean starting weight was 4.035 kilograms. They were housed twelve to an 8 x 12 foot pen containing 3 inch litter. The environment was semi-controlled: temperature was 60-70°F with continuous lighting. Chickens received feed (Agway Country Chick starter mash 2970 Kcal Me/kg, composition: crude protein - 18%, fat - 3%, fiber - 4%), and water, ad libitum.

RECOMBINANT CHICKEN GROWTH HORMONE

The hormone was supplied by American Cyanamid.

OSMOTIC PUMPS

Alzet osmotic pump implants (model 2m12, Alza Corp., Palo Alto, California - calibrated to deliver 2.5 microliters/hr/30 days) were used to administer rcGH. The osmotic pumps were treated as described above. The control group received saline vehicle (pH 8.4) only. The low and high dose experimental groups received 5 micrograms/kg/day rcGH and 50 micrograms/kg/day rcGH in saline (pH 8.4) respectively. Each pump was filled with 2 mls of designated solution and inserted subcutaneously over the scapula. The

6

wounds were closed with metal clips.

GROWTH PARAMETERS

Body weights were measured at 12 weeks, 13 weeks, 14 weeks, and 15 weeks of age, corresponding to day 0, 7, 14, and 21 of the experiment. The change in body weights each week was calculated. Shank-toe length was measured on the last day.

SAMPLES TAKEN

Immediately before killing each animal, 10 mls of blood were taken from the jugular vein and mixed with heparin. Samples were then placed on ice until all were centrifuged. The plasma was kept frozen until needed for glucose assays.

The chickens were killed by decapitation. Carcasses were dissected and the following samples were removed: the right pectoral muscle, abdominal adipose tissue, guts, heart, liver, and testes glands.

STATISTICAL ANALYSIS

The means of all results were assessed for significance through analysis of variance (one way ANOVA) within the groups and the range test (Least Significant Differential).

TABLE 1

| Effect of rcGH on Organ Weights in Chickens | | |
|---|---|---|
| Organ | Treatment Groups [a] | |
| | Saline (n = 9) | 5 micrograms/kg/day (n = 10) | 50 micrograms/kg/day (n = 8) |
| Muscle | 314±14 | 333±18 | 378±16* |
| Adipose | 54.4±12.3 | 81.8±10.1 | 103.9±15.3* |
| Guts | 333.6±26.3 | 311.7±21.6 | 302.2±6.5 |
| Heart | 26.7±1.9 | 27.4±1.1 | 25.7±1.4 |
| Liver | 78.5±3.7 | 73.5±2.7 | 75.2±2.4 |
| Testes | 32.4±3.1 | 33.8±5.5 | 28.5±4.7 |
| Mean weights in grams. | | |

*P.05
Different from controls
**P.01
[a]Treatments (continuously administered via Alzet[R] osmotic pump) for 21 days beginning 12 weeks old.

TABLE 2

| Effect of Recombinant Chicken GH on Growth | | | |
|---|---|---|---|
| | 0 (n = 9) | 5 micrograms/kg/day (n = 10) | 50 micrograms/kg/day (n = 8) |
| Initial BW (kg) (Age - 12 wks) | 4.05±0.12 | 4.11±0.10 | 4.17±0.08 |
| Final BW (kg) (Age - 15 wks) | 4.69±0.13 | 5.00±.09 | 5.22±0.11** |
| Average Daily Gain (g) | 30.4±6.1 | 43.1±2.9 | 47.9±5.0** |
| Muscle wt (g) (pectoralis) | 314±14 | 333±18 | 378±16* |
| Adipose wt (g) (abdominal) | 54.4±12.3 | 81.8±10.1 | 103.9±15.3** |

*p < 0.05
**p < 0.01

7

It will be noted that in use of 5 micrograms of GH per day, Average Daily Gain was increased by 41.8 percent:

$$\frac{43.1 - 30.4}{30.4} \times 100$$

and in use of 50 micrograms per day, Average Daily Gain was increased by 57.6 percent:

$$\frac{47.9 - 30.4}{30.4} \times 100$$

This is in great and unexpected contrast to the conclusion of W. H. Burke et al. in an article published in Endocrinology, Vol. 120, No. 2, 651-658 (1987), wherein the following opposite conclusion was reached with regard to administration of GH to broiler chickens rather than roaster chickens to which this invention is directed:

"Within 60 min. after sc injection or rcGH (480-960 micrograms/kg) in chickens, plasma GH levels increased 4- to 6-fold and remained significantly elevated for at least 5 h. Thrice-daily injections from age 2-24 days had little effect on growth or feed consumption in either male or female broiler chicks . . . The reduced rcGH had no effect on carcass protein, ash content, or nitrogen retention . . . This study shows that administration of rcGH to chickens can lead to some significant metabolic effects. However, it is the conclusion of this report that the level of circulating GH is not the limiting factor in the growth of this highly selected species."

Example 2

Five replicates of 5 birds (cockerels) each were inoculated at 8 weeks of age with 50 or 250 micrograms/kg/day of cST daily for 21 days, or 1 time with the equivalent of 150 micrograms/kg/day of a sustained release formulation of bST. Appropriate normal and sham-inoculated controls were included. The individual treatment replicates were maintained in floor pens for 4 weeks. Weekly weights were taken, and feed usage was monitored continuously. The birds were sacrificed at termination, and treatment effects on carcass quality, weight gain, and feed efficiency were determined.

The treatments had no effect on the 4 week weight gain (P = 0.7356) or on the feed efficiency (feed/gain; P = 0.6141; Table 1). Birds receiving 250 micrograms/kg/day of cST had numerically higher values for carcass yield, breast yield, and liver and gizzard weights, and lower fat pad weights, than the normal controls or any of the other treatment groups. These values were not significantly different from the normal controls (Table 2).

When compared with the appropriate sham-inoculated control (Table 2), breast yield was significantly improved (P = 0.0078) after adjustment for starting bird weight, in the groups receiving 250 micrograms/kg/day of cST, but none of the other measurements were significantly affected. Shank lengths of the birds receiving 150 micrograms/kg/day of bST were significantly reduced (P = 0.0147).

Free fatty acid levels were significantly reduced at 250 micrograms/kg/day compared to control but were not reduced compared to sham-inoculated control (Table 2).

## TABLE 3

cST-A-88-7
Weight Gain and Feed Efficiency (F-G) - 0-4 Weeks

| | | |
|---|---|---|
| N-Control | 1.77 | 4.48 |
| cST-50 | 1.61 | 4.74 |
| cST-250 | 1.82 | 4.19 |
| cST-Control | 1.82 | 4.08 |
| bST-Control | 1.73 | 4.33 |
| bST-150 | 1.69 | 4.54 |

## TABLE 4

cST-a-88-7
Carcass Quality - % of Live Weight of N-Control
- Adjusted for Starting Weight of Birds

| | Carcass Yield | Breast Yield | Fat Pad | Liver | Giz- zard | Heart | FFA |
|---|---|---|---|---|---|---|---|
| N-Control | 71.02 | 21.68 | 3.09 | 1.50 | 0.936 | 0.411 | 464.2 |
| cST-50 | 71.11 | 21.24 | 2.60 | 1.44 | 0.841 | 0.409 | 408.6 |
| cST-250 | 73.36 | 23.47** | 2.36 | 1.39 | 0.808 | 0.418 | 339.0 |
| cST-Control | 71.63 | 21.76 | 2.63 | 1.49 | 0.917 | 0.449 | 363.6 |
| bST-Control | 71.23 | 21.54 | 2.56 | 1.53 | 0.875 | 0.409 | 305.5 |
| bST-150 | 68.47 | 20.35 | 2.83 | 1.62 | 0.916 | 0.395 | 358.5 |

** $P < .05$

FFA = blood free fatty acids

Example 3

Preparation of injectable microspheres for the parenteral administration of recombinant animal somatotropin derivatives

Preparation of the novel recombinant animal somatotropins in a size range suitable for incorporation in microspheres by spray drying is accomplished by dissolving the recombinant animal somatotropin derivative in dilute ammonium hydroxide solution and then adding desired salt solutions such as sodium benzoate. A nonionic surfactant such as a block copolymer of ethylene oxide and propylene oxide is added and allowed to dissolve with constant gentle mixing. The solution is then spray-dried. A Buchi mini spray dryer, model #190 maybe used for this purpose.

A homogeneous mixture of the thus prepared active ingredient and additives in the molten fat, wax or mixture thereof is prepared and the resulting mixture sprayed through an air/liquid spray nozzle equipped with a heated jacket to maintain the incoming air and the molten phase at a temperature above the melting point. The microspheres are formed as the molten droplets cool and are collected on a series of sleves in the desired size range of about 45 to 180 microns and retained for use. Microspheres which are not of the desired size range are collected for recycling. Alternatively, the homogeneous mixture are fed onto a centrifugal disc and the microspheres thus formed are collected as above, or the molten mixtures are cooled and milled to the desired average particle size range.

Waxes and fats which are suitable for use in the compositions of this invention in general have melting points higher than 40°C. *These waxes are defined as a low-melting organic mixture or compound of high molecular weight, solid at room temperature and generally similar in composition to fats and oils except that it contains no glycerides. Some are hydrocarbons; others are esters of fatty acids and alcohols. They are classed among the lipids. Waxes are thermoplastic, but since they are not high polymers, they are not considered in the family of plastics. Common properties are water repellency, smooth texture; nontoxicity; freedom from objectionable odor and color. They are combustible, and have good dielectric properties. Soluble in most organic solvents; insoluble in water. The major types are as follows:

I. Natural

    1. Animal (beeswax, lanolin, shellac wax, Chinese insect wax).
    2. Vegetable (carnauba, candelilla, bayberry, sugar cane)
    3. Mineral
        (a) Fossil or earth waxes (ozocerite, ceresin, montan)
        (b) petroleum waxes (paraffin, microcrystalline) (slack or scale wax)

II. Synthetic

    1. Ethylenic polymers and polyol ether-esters ("Carbowax," sorbitol)
    2. Chlorinated naphthalenes ("Halowax")
    3. Hydrocarbon type via Ficher-Tropsch synthesis

The fat of the invention may be defined as a glyceryl ester of higher fatty acids such as stearic and palmitic. Such esters and their mixtures are solids at room temperatures and exhibit crystalline structure. Lard and tallow are examples. There is no chemical difference between a fat and an oil, the only distinction being that fats are solid at room temperature and oils are liquid. The term "fat" usually refers to triglycerides specifically, whereas "lipid" is all-inclusive.

The fat is preferably long chain $C_{10}$-$C_{24}$ fatty acid, alcohol, ester, salt, ether or mixture thereof, with mono-, di-, or triglycerides composed predominantly of stearates, palmitates, laurates, linoleates, linolenates, oleates, and residues or mixtures thereof, having melting points greater than 50°C being most preferred. Glycerol tristearate is a most preferred fat. Additionally, lipophilic salts of fatty acids such as magnesium stearate and the like are also suitable.

The microspheres of the invention are dispersed in a pharmaceutically and pharmacologically acceptable liquid to obtain a slow release composition for parenteral administration. The vehicle is aqueous buffered systems or oil systems. The oil is a vegetable or an animal oil. A preferred oil is a neutral triglyceride liquid fat. A neutral oil is one containing no residual acid. Vehicles suitable for use in the compositions of this invention include aqueous systems such as buffered salines; organic solvents such as glycols and alcohols; and water immiscible liquids such as oils, depending upon the solubility of the active ingredient being administered. Data obtained are reported in TABLE 5.

* The Condensed Chemical Dictionary
Tenth Edition Pg. 1094
Van Nostrand Reinhold Publisher

### TABLE 5

Microsphere formulations of recombinant animal somatotropin

| recombinant animal % somatotropin derivative | % cholic acid | Core % deoxy-cholic acid | % cholesterol | Coating % cholesterol | % glyceryl tristearate | % PVP | % beeswax | % phosphati-dylcholine |
|---|---|---|---|---|---|---|---|---|
| 50 | 50 | - | - | 42.5 | 42.5 | 5 | - | - |
| 20 | 80 | - | - | 42.5 | 42.5 | 5 | - | - |
| 75 | 25 | - | - | 42.5 | 42.5 | 5 | - | - |
| 50 | - | 50 | - | 40 | - | 5 | 40 | 5 |
| 20 | - | 80 | - | 40 | - | 5 | 40 | 5 |
| 75 | - | 25 | - | 40 | - | 5 | 40 | 5 |
| 50 | - | - | 50 | 42.5 | 42.5 | 5 | - | - |
| 20 | - | - | 20 | 42.5 | 42.5 | 5 | - | - |
| 75 | - | - | 75 | 42.5 | 42.5 | 5 | - | - |

EP 0 353 045 B1

## TABLE 6

### Microsphere Compositions

| Composition # | Matrix | rPST Derivatives | Additives(%) Buffer/Pres. | Additives(%) Surfactant |
|---|---|---|---|---|
| 1 | GMS | 2.5 | -- | -- |
| 2 | | 20 | -- | -- |
| 3 | GMS/soya oil | 30 | -- | -- |
| 4 | GDS | 10 | -- | -- |
| 5 | | 10 | NaLaurate(1.4) | -- |
| 6 | | 25 | NaBenzoate(1.6) | Surf.B(0.12) |
| 7 | | 25 | NaCarb/bicarb(1.3) | Surf.A(0.18) |
| 8 | GTS/GDS (19:1) | 15 | -- | -- |
| 9 | | 10 | NaBenzoate(0.6) | Surf.B(0.05) |
| 10 | GTS/GDS (9:1) | 10 | -- | Surf.B(0.1) |
| 11 | GTS/beeswax (9:1) | 7.5 | -- | -- |
| 12 | GTS/GMS (19:1) | 15 | NaBenzoate (1.4) | -- |
| 13 | GTS/Soya oil (19:1) | | 7.5 | -- -- |
| 14 | GMS/Carnauba (1:2) | 20 | -- | -- |
| 15 | | 10 | NaBenzoate(0.3) | -- |

EP 0 353 045 B1

TABLE 6 (Continued)

Microsphere Compositions

| Composition # | Matrix | rPST Derivatives | Additives(%) Buffer/Pres. | Surfactant |
|---|---|---|---|---|
| 16 | | 10 | -- | Surf.B(0.05) |
| 17 | | 25 | NaBenzoate(0.75) | Surf.B(0.12) |
| 18 | | 25 | NaBenzoate(3.2) | Surf.B(0.12) |
| 19 | | 25 | -- | Surf.B(0.12) |
| 20 | | 25 | -- | Surf.A(0.48) |
| 21 | | 25 | NaCarb/bicarb(1.2) | -- |
| 22 | GTL | 33 | NaBenzoate(2.1) | Surf.A(0.17) |
| 23 | GTS/GDS(12:1) | 15 | CaBenzoate(1.1) | -- |
| 24 | Beeswax/Soya Oil (2:1) | 30 | -- | -- |
| 25 | GTS | 7.5 | -- | -- |
| 26 | GMS/Soya Oil (47.25/24) | 25 | -- | Sorbitan |
| 27 | | 15 | -- | monoleate (3.75) |
| 28 | GMS | 10 | -- | POE(23) stearate (9.0) |

EP 0 353 045 B1

## TABLE 6 (Continued)

## Microsphere Compositions

| Composition # | Matrix | rPST Derivatives | Additives(%) Buffer/Pres. | Surfactant |
|---|---|---|---|---|
| 29 | GMS | 20 | -- | PEG 6000(9.0) |
| 30 | GMS | 20 | -- | PEG 6000(4.0) |
| 31 | GMS | 20 | -- | PEG 6000(0.8) |
| 32 | GMS | 20 | -- | PEG 400 distearate (8.0) |
| 33 | GMS | 20 | -- | PEG 400 distearate (1.57) |
| 34 | GMS | 20 | -- | PEG 1540 distearate (16.0) |
| 35 | Diethylene glycol monostearate | 25 | -- | -- |

Example 4 (Not within the scope of the claims).

Bile Acid Microspheres

A buffered solution of rpST derivative and bile acid is spray dried in a Buchi Model 190 spray dryer. This fine particle powder (10 parts) is suspended in a methylene chloride solution of coating materials (1 part). This suspension is spray dried. The coated microspheres are suspended in a suitable suspending vehicle prior to injection. These compositions are described in Table II. Other derivatized or modified recombinant animal somatotropins of the present invention, including the modified recombinant avian somatotropins, wherein the cysteines in the small loop at the 183 and 191 positions (184 and 191 for humans) of the recombinant animal somatotrophins are replaced by alanine, serine, glutamic acid, arginine, lysine or the like, are prepared as microspheres in accordance with the description in TABLE 6.

Example 5 (Not within the scope of the claims).

Preparation of recombinant animal somatotropin implants

Implants are prepared by weighing a sufficient quantity of the ground homogeneous mixture of the desired recombinant animal somatotropin derivative and the desired diluents. This mixture is then compressed on a carver press at from (1000 x 6.89) + 101 - (6000 x 6.89) + 101 kPa (1000 to 5000 psig) in a (3/16" or 1/8") 3 x 25.4/16 - 25.4/8 mm diameter cylindrical die or on a rotary tablet press using the required punch and die. The implants thus prepared are then coated with either biodegradable or nonbiodegradable coatings by procedures A and B.

Procedure A

Non-Biodegradable Silicon Polymer

Clean grade silicon elastomer (10 parts) is mixed with curing agent (one part) on a watch glass with a spatula. This is deaerated in a dessicator for 30 minutes. The implants are grasped by the ends with tweezers, rolled into the silicon polymer, placed on end on aluminum foil and cured at 40°C for five hours. One or both of the ends are removed with a razor blade leaving the "shaft" of the cylinder coated.

Alternatively, implants are dip coated with 20% to 40% of a medical adhesive, sold under the trademark SILASTIC® by Dow Corning, which has been dispersed in hexane, and dried and cured at 40°C to 50°C overnight before removing the coating from one or both of the base ends.

Procedure B

Biodegradable Coatings

The polymer or copolymer (one part) is dissolved in chloroform (three to eight parts). Each implant is grasped by the ends with tweezers, dipped into the polymer solution, and then the chloroform evaporated at room temperature. Each implant is coated twice. After the coating dried overnight at room temperature, the polymer ends are removed with a razor blade, leaving the long cylindrical "shaft" coated.

## Implant formulation

| % recombinant porcine somatotropin derivative | % Magnesium stearate | % ethyl cellulose | % Castor Wax |
|---|---|---|---|
| 50 | – | 5.0 | 45 |
| 40 | – | 5.0 | 50 |
| 20 | – | 5.0 | 75 |
| 50 | 0.5 | 3.5 | 46 |
| 20 | 0.5 | 3.5 | 76 |

| % rpST derivative | % cholesterol | % surfactant | % glyceryl tristearate |
|---|---|---|---|
| 30 | 68 | 2 | – |
| 15 | 41.5 | ·2 | 41.5 |

| % rpST derivative | % stearic acid |
|---|---|
| 50 | 50 |
| 20 | 80 |

Alternatively, rpST or other recombinant animal somatotropins is blended with surfactants, buffer salts, and/or preservatives in an aqueous solution. This solution is then spray-dried in a Buchi Model 190 spray dryer giving a small particle size powder. This powder is then melt-blended with a fat or wax and molded into cylindrical implants. The implants prepared above are then coated with either a biodegradable or a non-degradable polymer using procedure A or B.

| Implant Formulations | | | |
|---|---|---|---|
| % rpST derivative | % glyceryl tristearate | % sodium benzoate | % surfactant |
| 28 | 69.9 | 2.0 | 0.15 |
| 15 | 82.9 | 2.0 | 0.15 |
| 50 | 48.5 | 1.5 | 0 |

Example 6 (Not within the scope of the claims).

Preparation of implants using (Cam-Cys 183,191) rpST and evaluation of said implants by in vitro dissolution

To 1.3 ml of $CH_2Cl_2$ is added 92.4 ms of co-poly (glycolide/lactide) copolymer, 5.3 mg of a nonionic block copolymer of propylene oxide and ethylene oxide marketed by BASF Wyandottee Corp. as pluronic 127 which has an average molecular weight of 12,500, m.p. 56 and Brookfield viscosity of 3100 (cps)[35], 5.3

mg of 200 mesh mg $(OH)_2$ and 74.6 mg of powdered (Cam-Cys 183,191)rpST. The mixture is agitated, poured over a large surface petri dish and the $CH_2Cl_2$ allowed to evaporate at room temperature and then dried by vacuum drying. The dried residue is collected and formed in (1/8") 25.4/8 mm diameter cylindrical implants using a Carver Press at about 1000 psig. The thus formed implants weigh 60 to 70 mg each and are designated I-1.

A second set of implants are prepared by mixing together in a Vortex-genie mixer 128 mg of powdered castorwax (-270 mesh) and 32 mg of powdered (Cam-Cys 183,191)rpST. The thus prepared mixture is then formed into (1/8") 25.4/8 mm diameter cylindrical implants in the manner described above. The pellets weigh 60 to 70 mg each and are designated I-2.

A third set of implants are also prepared by the procedures described above using 96 mg of -270 mesh castorwax and 64 mg of powdered (Cam-Cys 183,191)rPST. The (1/8") 25.4/8 mm diameter cylindrical implants prepared as descried above weigh 60 to 70 mg and are designated I-3.

The thus prepared implants are then subjected to an in vitro dissolution evaluation. Each of the implants is placed in a separate plastic tube containing 10 ml of a phosphate buffer solution (ph 7.4 with 0.05% Na azide) and the tubes placed in a shaking water rack where the tubes are shaken while the temperature of the water in the unit is maintained at 39°C. The tubes are shaken for one day then the solutions removed from each tube and analyzed for rpST by HPLC and the solution discarded. New phosphate buffer solution is added to each tube and the tubes shaken for three additional days thereafter. The solutions from each tube is again analyzed for rpST by HPLC and the solution again discarded. New phosphate buffer is again added to each tube and the tube again shaken for three days then analyzed again for rpST.

The implants used in these determinations are described below along with the results obtained. The phosphate buffer solution is ($NaH_2PO_4 \cdot H_2O$ 3.45 g, $Na_2HPO_4$ 3.55 g, Nacl 9.5 g dissolved in distilled water to 1000 ml.

| Implant Preparation and Designation | | |
|---|---|---|
| rpST in Implant Origin of Implant | Wt. of Implant | (Theory) mg |
| I-1 | 69.6 mg | 29.23 mg |
| I-2 | 68.7 mg | 28.85 mg |
| I-3 | 62.8 mg | 12.56 mg |
| I-4 | 63.3 mg | 12.66 mg |
| I-5 | 66.8 mg | 26.72 mg |
| I-6 | 66.2 mg | 26.48 mg |

| In Vitro Dissolution Results | | | | | | | |
|---|---|---|---|---|---|---|---|
| Vol of release media (ml) | Day | I-1 % Dimer | I-2 % Dimer | I-3 % Dimer | I-4 % Dimer | I-5 % Dimer | I-6 % Dimer |
| 10 | - | Start | Start | Start | Start | Start | Start |
| 10 | 1 | 1.59 (low) | 1.184 (1.3%) | 0.485 (low, not measured | 0.499 (low, not measured | 1.605 (low) | 1.570 (low) |
| 10 | 4 | 0.417 (low) | 0.459 (1.7%) | 0.063 (low) | 0.068 (low) | 0.136 (low) | 0.140 (low) |
| 10 | 7 | 0.044 (low, not measured | 0.038 (low, not measured | 0.007 (low, not measured | 0.007 (low not measured | 0.019 (low, not measured | 0.019 (low, not measured |

## Cumulative Release of rPST

### I-1 plus I-2

| Day | Cumulative mg. released | Cumulative % of orignal released |
|---|---|---|
| 1 | 11.72 | 40.4% |
| 4 | 16.10 | 55.4% |
| 7 | 16.51 | 56.9% |

### I-3 plus I-4

| | | |
|---|---|---|
| 1 | 4.92 | 39.02% |
| 4 | 5.58 | 44.25% |
| 7 | 5.65 | 44.81% |

### I-5 plus I-6

| | | |
|---|---|---|
| 1 | 15.88 | 59.7 |
| 4 | 17.26 | 64.89% |
| 7 | 17.45 | 65.6 % |

Example 7 (Not within the scope of the claims).

Preparation of microencapsulated recombinant animal somatotropin

Poly(lactide-co-glycolide), 0.85 g, is dissolved in 29.8 g of chloroform and vortexed for 2 minutes. A recombinant porcine somatotropin derivative (Cam-Cys 183,191)rpST, 0.150 g, which has been dry sieved thru a 25-micron stainless steel sieve, is then added to the polymer/chloroform solution and vortexed for 3 minutes. The resulting suspension of the protein is then charged to a 50-ml round bottom vessel filled with a mechanical stirrer. The stir speed is set at 700 rpm at ambient temperature. An additional 3.2 g of chloroform is used to rinse the initial vessel. Then, the total chloroform quantity is 32.95 g. Silicone oil (350 cs) is then added (13.1 g over a period of 8 minutes) to precipitate the poly(lactide- co-glycolide). The contents of the flask are transferred to 1720 g of heptane with mechnaical stirring in a 4-liter flask. After 3 hours, the hardened microspheres are filtered through a series of stainless-steel sieves and dried under vacuum.

Other polymers and solvents useful for microencopsulation of modified recombinant animal somatotropins wherein one or more of the cysteine, amino acid residues at the 55, 166, 183 or 191 positions of the recombinant animal is replaced by a different amino acid residue or a derivatized recombinant in which one or both of the sulfide bridges between the cysteines at the 55 and 166 positions or between the 183 and 191 positions is reduced and derivatized, are listed below.

Abbreviations used in the listing of materials set forth below are:

A      = as polymerized sample
B      = reprecipitated sample
gly    = glycolide

lact     = lactide
capro    = caprolactone
TMC      = trimethylene carbonate
PHB      = polyhydroxybutyrate
PHV      = polyhydroxyvalerate
PEO      = polyethylene oxide

| Polymers useful for Microencapsulation of modified and derivatized recombinant animal somatotropin | | |
|---|---|---|
| Polymer | Ninh (Solvent) | Compositions (Wgt. %) |
| Gly/dl-lactide | $0.36^A$ (HFIP) | $43.6/56.4^A$ |
| Gly/dl-lactide | $0.52^A$ (HFIP) | $42.4/57.4^A$ |
| Gly/dl-lactide | $0.63^B$ (HFIP) | $43.3/56.7^B$ |
| Gly/dl-lactide | $0.66^B$ (HFIP) | $41.2/58.8^B$ |
| Gly/dl-lactide | $0.61^A$ (HFIP) | $41.6/58.4^A$ |
| Gly/dl-lactide | $0.22^B$ (HFIP) | $41.5/58.5^B$ |
| Gly/dl-lactide | $0.26^B$ (HFIP) | $40.6/59.4^B$ |
| Gly/dl-lactide | $0.27^B$ (HFIP) | $40.7/59.3^B$ |
| Gly/L-lact | $0.2^B$ ($CHCl_3$) | $48/52^B$ |
| dl-lact | $0.46^B$ (HFIP) | $100^B$ |
| dl-lact | $0.27^B$ (HFIP) | $100^B$ |
| PHB | $2.92^A$ (HFAS) | $100^A$ |
| PHB/HV | $3.27^A$ (HFAS) | $70/30^A$ |
| Capro/l-lact | $0.45^A$ ($CHCl_3$) | $84.6/15.4^A$ |
| Capro/l-lact | $0.46^A$ ($CHCl_3$) | $84.0/16.0^A$ |
| Capro/l-lact | $0.51^A$ ($CHCl_3$) | $85.2/14.8^A$ |
| Capro/l-lact | $0.50^A$ ($CHCl_3$) | $84.4/15.6^A$ |
| Capro/l-lact | $0.39^A$ ($CHCl_3$) | $85.1/14.9^A$ |
| Capro/l-lact | $0.36^A$ ($CHCl_3$) | $86.1/13.9^A$ |
| Capro/l-lact | $0.31^B$ ($CHCl_3$) | $11.4/8.6^B$ |
| Capro/gly | $0.34^A$ ($CHCl_3$) | $84.0/16.0^A$ |
| gly/PEO 8,000/TMC | $0.33^B$ ($CHCl_3$) | $50.3/8.4/41.3^B$ |
| gly/PEO 8,000/TMC | $0.38^B$ ($CHCl_3$) | $58.2/4.8/37.0^B$ |
| gly/dl-lactide/PEO 8,000 | $0.35^B$ ($CHCl_3$) | $35.8/54.4/9.8^B$ |
| 1-lact/TMC | $0.26^B$ ($CHCl_3$) | $85.4/14.6^B$ |
|  | $0.23^B$ ($CHCl_3$) | $69/31^B$ |

A) = as polymerized sample
B) = reprecipitated sample

### Claims

1. A biocompatible subcutaneous implant for use in promoting growth or feed efficiency in roasters, said implant containing an avian somatotropin and said implant being capable of delivering continuously for a predetermined minimum time of at least 14 days said somatotropin for systemic absorption in a predetermined amount of at least about 1 microgram/kg of said roasters/day.

2. An implant as claimed in claim 1 containing recombinant chicken somatotropin.

3. An implant as claimed in claim 1 or claim 2 which is capable of delivering at least about 5 micrograms/kg of said roasters/day for at least 21 days.

4. An implant as claimed in any of the preceding claims which is capable of delivering at least about 50 micrograms/kg of said roasters/day for at least 21 days.

5. An implant as claimed in any of the preceding claims in the form of a pellet.

EP 0 353 045 B1

6. A method for increasing the rate of growth or feed efficiency of roasters, which method comprises parenterally administering to said roasters by continuous infusion for a minimum time of at least 14 days a growth promoting amount of at least about 1 microgram/kg of said roasters/day of a somatotropin.

7. A method as claimed in claim 6 wherein the somatotropin is administered using an implant as claimed in any of claims 1 to 5.

8. A method as claimed in claim 6 wherein the somatotropin is administered as a subcutaneous implant or a sustained release formulation.

9. A pharmaceutical composition for parenteral administration comprising an avian somatotropin or a pharmaceutically acceptable salt thereof together with a pharmaceutically acceptable solid or liquid carrier medium, wherein said composition is adapted to deliver continuously for a predetermined minimum time of at least 14 days said somatotropin for systemic absorption in a predetermined amount of at least about 1 microgram/kg of said roaster/day, and said composition is effective in increasing the growth rate of a roaster to which said composition is administered parenterally over an extended period of time.

**Patentansprüche**

1. Biokompatibles, subkutanes Implantat zur Verwendung zur Förderung des Wachstums oder der Futtereffizienz bei Ferkeln, wobei das Implantat ein Vogelsomatotropin enthält und des Somatotropin kontinuierlich über eine vorbestimmte Minimalzeit von mindestens 14 Tagen zur systemischen Absorption in einer vorbestimmten Menge von mindestens etwa 1 µg/kg des Ferkels/Tag abgeben kann.

2. Implantat nach Anspruch 1, enthaltend rekombinantes Hühnersomatotropin.

3. Implantat nach Anspruch 1 oder 2, welches mindestens etwa 5 µg/kg des Ferkels/Tag für mindestens 21 Tage abgeben kann.

4. Implantat nach einem der vorausgehenden Ansprüche, welches mindestens etwa 50 µg/kg des Ferkels/Tag für mindestens 21 Tage abgeben kann.

5. Implantat nach einem der vorausgehenden Ansprüche in Form eines Pellets.

6. Verfahren zur Erhöhung der Wachstumsrate oder der Futtereffizienz bei Ferkeln, wobei man den Ferkeln durch kontinuierliche Infusion über eine Minimalzeit von mindestens 14 Tagen eine das Wachstum fördernde Menge von mindestens etwa 1 µg/kg des Ferkels/Tag eines Somatotropins parenteral verabreicht.

7. Verfahren nach Anspruch 6, wobei man das Somatotropin unter Verwendung eines Implantats nach einem der Ansprüche 1 bis 5 verabreicht.

8. Verfahren nach Anspruch 6, wobei man das Somatotropin als subkutanes Implantat oder als Formulierung mit verzögerter Freisetzung verabreicht.

9. Pharmazeutische Zusammensetzung für die parenterale Verabreichung, umfassend ein Vogelsomatotropin oder ein pharmazeutisch verträgliches Satz davon zusammen mit einem pharmazeutisch verträglichen festen oder flüssigen Trägermedium, wobei die Zusammensetzung der kontinuierlichen Abgabe des Somatotropins über eine vorbestimmte Minimalzeit von mindestens 14 Tagen zur systemischen Absorption in einer vorbestimmten Menge von mindestens etwa 1 µg/kg des Ferkels angepaßt ist, wobei die Zusammensetzung effektiv die Wachstumsrate eines Ferkels, dem die Zusammensetzung parenteral über eine längere Zeitspanne verabreicht wird, erhöht.

20

**Revendications**

1. Implant sous-cutané biocompatible destiné à favoriser la croissance et l'engraissage des volailles, ledit implant contenant une hormone de croissance aviaire et ledit implant étant apte à libérer de façon continue sur une période minimale prédéterminée d'au moins 14 jours, ladite hormone de croissance en vue d'une absorption systémique dans une quantité prédéterminée d'au moins environ 1 μg/kg de volaille/jour.

2. Implant selon la revendication 1, contenant une hormone de croissance recombinante provenant de volailles.

3. Implant selon la revendication 1 ou la revendication 2, apte à libérer au moins environ 5 μg/kg de volaille/jour pendant au moins 21 jours.

4. Implant selon l'une quelconque des revendications précédentes, apte à libérer environ 50 μg/kg de volaille/jour pendant au moins 21 jours.

5. Implant selon l'une quelconque des revendications précédentes, sous la forme d'une pastille.

6. Procédé pour favoriser le taux de croissance et d'engraissage des volailles, ledit procédé comprenant l'administration par voie parentérale d'une hormone de croissance à ladite volaille par perfusion en continu sur une période minimale d'au moins 14 jours d'une quantité apte à favoriser la croissance d'au moins environ 1 μg/kg de volaille/jour.

7. Procédé selon la revendication 6 dans lequel l'hormone de croissance est administrée en utilisant un implant selon l'une quelconque des revendications 1 à 5.

8. Procédé selon la revendication 6 dans lequel l'hormone de croissance est administrée sous la forme d'un implant sous-cutané ou d'une préparation à libération prolongée.

9. Composition pharmaceutique pour l'administration parentérale comprenant une hormone de croissance aviaire ou un sel pharmaceutiquement acceptable de celle-ci associée à un véhicule solide ou liquide pharmaceutiquement acceptable, ladite composition étant apte à libérer de façon continue sur une période minimale prédéterminée d'au moins 14 jours ladite hormone de croissance en vue d'une absorption systémique dans une quantité prédéterminée d'au moins environ 1 μg/kg de volaille/jour, et ladite composition étant apte à augmenter le taux de croissance d'une volaille à laquelle ladite composition est administrée par voie parentérale sur une période prolongée.

F I G. 1